# EUROPEAN PATENT APPLICATION

(11) **EP 3 838 126 A1**
(43) Date of publication of application: **23.06.2021**
(21) Application number: 18930322.5
(22) Date of filing: 17.08.2018
(51) Int. Cl.: A61B 5/0205, G06Q 50/22

(54) **BLOOD OXYGEN SATURATION-BASED EVALUATION METHOD AND APPARATUS, INTELLIGENT WEARABLE DEVICE AND STORAGE MEDIUM**

(71) Applicant: Coros Sports Technology (Shenzhen) Co., Ltd, Qianhai Shenzhen, Guangdong 518000 (CN)
(72) Inventor: LIU, Xin, Shenzhen, Guangdong 518000 (CN); WANG, Xiaohu, Shenzhen, Guangdong 518000 (CN); ZHAO, Wenliang, Shenzhen, Guangdong 518000 (CN); TANG, Yu, Shenzhen, Guangdong 518000 (CN); ZUO, Hailiang, Shenzhen, Guangdong 518000 (CN)
(74) Representative: Goddar, Heinz J.
(86) International application number: PCT/CN2018/101092
(87) International publication number: WO 2020/034198

(57) **Abstract**

Provided is an oxygen saturation-based assessment method, the method includes: obtaining an elevation and oxygen saturation corresponding to a user to be assessed, determining an assessment result corresponding to the user to be assessed based on the elevation and the oxygen saturation. The assessment method can timely assess the adaptability of the user to be assessed, which can prevent the occurrence of plateau reactions and plateau disease in advance and provide exercise guidance. And an oxygen saturation-based assessment device, smart wearable device and storage medium are provided further.

## Description

### CROSS-REFERENCE TO RELATED APPLICATION

This application is a continuation of International Application No. PCT/CN2018/101092, filed August 17, 2018, the entire content of which is incorporated herein by reference.

### TECHNICAL FIELD

This disclosure relates to the fields of health assessment and smart wearable device, and particularly to an oxygen saturation-based assessment method, device, smart wearable device and storage medium.

### BACKGROUND

In recent years, the activity of trekking across the Sichuan-Tibet route has emerged, and more and more people like to go to plateau areas such as Tibet for hiking. However, plateau area is dangerous for people who have lived in plain for a long time, because the air pressure is low and the oxygen content is thin, people who do not adapt to it often have plateau reaction which can be life-threatening in serious cases.

Plateau reaction is an acute plateau sickness, which is a series of symptoms and metabolic changes caused by the change in air pressure, oxygen content, dry air, cold, strong ultraviolet rays, after a person reaches a certain elevation (generally referred to elevation above 3000 meters).

### SUMMARY

### TECHNICAL PROBLEM

However, there are currently only a few remedy and preventive drugs for plateau reaction, such as oxygen inhalers, Chinese medicine, etc., and it is not possible to assess the plateau adaptability in time. Based on this, it is necessary to address the above problems and provide an oxygen saturation-based assessment method, device, smart wearable device, and storage medium that can assess plateau adaptability in time.

In accordance with a first aspect of the present disclosure, an oxygen saturation-base method is provided. The method includes:

Obtaining an elevation and oxygen saturation corresponding to a user to be assessed;

Determining an assessment result corresponding to the user to be assessed based on the elevation and the oxygen saturation.

In one embodiment, obtaining the elevation and the oxygen saturation corresponding to the user to be assessed includes obtaining a predicted elevation corresponding to the user to be assessed. Determining the assessment result corresponding to the user to be assessed based on the elevation and the oxygen saturation includes calculating a rate of change of oxygen saturation based on the oxygen saturation corresponding to the user to be assessed, and determining the assessment result corresponding to the user to be assessed based on the predicted elevation and the rate of change of oxygen saturation.

In one embodiment, obtaining the elevation and the oxygen saturation corresponding to the user to be assessed includes obtaining a current elevation and a current oxygen saturation corresponding to the user to be assessed, and obtaining a first oxygen saturation corresponding to the user to be assessed at a first elevation, the first elevation being less than the current elevation. Determining the assessment result corresponding to the user to be assessed based on the elevation and the oxygen saturation includes determining the assessment result corresponding to the user to be assessed at the current elevation based on the current oxygen saturation and the first oxygen saturation.

In one embodiment, the method further includes calculating a current oxygen saturation prewarning value based on the current elevation. Determining the assessment result corresponding to the user to be assessed at the current elevation based on the current oxygen saturation and the first oxygen saturation includes determining the assessment result corresponding to the user to be assessed based on the current oxygen saturation, the first oxygen saturation and the current oxygen saturation prewarning value.

In one embodiment, calculating the current oxygen saturation prewarning value based on the current elevation includes obtaining a correlation between elevation and oxygen saturation prewarning value, the correlation being obtained based on the oxygen saturation corresponding to plateau reaction and non-plateau reaction under different elevations, and calculating the current oxygen saturation prewarning value corresponding to the current elevation based on the correlation.

In one embodiment, determining the assessment result corresponding to the user to be assessed based on the current oxygen saturation, the first oxygen saturation and the current oxygen saturation prewarning value includes determining the assessment result corresponding to the user to be assessed as first adaptability level when the difference of the current oxygen saturation and the first oxygen saturation being greater than a predetermined threshold, and determining the assessment result corresponding to the user to be assessed as second adaptability level when the current oxygen saturation being less than the current oxygen saturation prewarning value and greater than the difference of the first oxygen saturation and the predetermined threshold.

In one embodiment, the method further includes obtaining a current exercise intensity corresponding to the user to be assessed, and calculating a current oxygen saturation standard value based on the current exercise intensity and the current elevation. Determining the assessment result corresponding to the user to be assessed based on the current oxygen saturation, the first oxygen saturation and the current oxygen saturation prewarning value includes determining the assessment result corresponding to the user to be assessed based on the current oxygen saturation, the first oxygen saturation, the current oxygen saturation prewarning value and the current oxygen saturation standard value.

In one embodiment, calculating the current oxygen saturation standard value based on the current exercise intensity and the current elevation includes obtaining a causation of elevation, exercise intensity and oxygen saturation standard value, the causation being obtained by analyzing oxygen saturation acquired under various elevations and exercise intensities, and calculating the current oxygen saturation standard value corresponding to the current exercise intensity and the current elevation based on the causation.

In one embodiment, determining the assessment result corresponding to the user to be assessed based on the current oxygen saturation, the first oxygen saturation, the current oxygen saturation prewarning value and the current oxygen saturation standard value includes determining the assessment result corresponding to the user to be assessed as third adaptability level when the current oxygen saturation being greater than the current oxygen saturation prewarning value and the current oxygen saturation standard value being less than the current oxygen saturation prewarning value, and determining the assessment result corresponding to the user to be assessed as fourth adaptability level when the current oxygen saturation being greater than the current oxygen saturation prewarning value and the current oxygen saturation standard value being no less than the current oxygen saturation prewarning value.

In one embodiment, the method further includes obtaining a predetermined oxygen saturation safety threshold and a predetermined exercise intensity, calculating a climbable elevation corresponding to the user to be assessed based on the current oxygen saturation, the predetermined oxygen saturation safety threshold and the predetermined exercise intensity.

An oxygen saturation-based assessment device, the device includes:

An obtaining module configured to obtain an elevation and oxygen saturation corresponding to a user to be assessed;

A determining module configured to determine an assessment result corresponding to the user to be assessed based on the elevation and the oxygen saturation.

In one embodiment, the obtaining module is further configured to obtain a predicted elevation corresponding to the user to be assessed. The determining module is further configured to calculate a rate of change of oxygen saturation based on the oxygen saturation corresponding to the user to be assessed, and determine the assessment result corresponding to the user to be assessed based on the predicted elevation and the rate of change of oxygen saturation.

In one embodiment, the obtaining module is further configured to obtain a current elevation and a current oxygen saturation corresponding to the user to be assessed, a first oxygen saturation corresponding to the user to be assessed at a first elevation, and the first elevation being less than the current elevation. The determining module is further configured to determine the assessment result corresponding to the user to be assessed at the current elevation based on the current oxygen saturation and the first oxygen saturation.

In one embodiment, the device further includes a prewarning value computation module configured to calculate a current oxygen saturation prewarning value based on the current elevation. The determining module is further configured to determine the assessment result corresponding to the user to be assessed based on the current oxygen saturation, the first oxygen saturation and the current oxygen saturation prewarning value.

In one embodiment, the prewarning value computation module is further configured to obtain obtaining a correlation between elevation and oxygen saturation prewarning value, the correlation being obtained based on the oxygen saturation corresponding to plateau reaction and non-plateau reaction under different elevations, and calculating the current oxygen saturation prewarning value corresponding to the current elevation based on the correlation.

In one embodiment, the determining module is further configured to determine the assessment result corresponding to the user to be assessed as first adaptability level when the difference of the current oxygen saturation and the first oxygen saturation being greater than a predetermined threshold, and determine the assessment result corresponding to the user to be assessed as second adaptability level when the current oxygen saturation being less than the current oxygen saturation prewarning value and greater than the difference of the first oxygen saturation and the predetermined threshold.

In one embodiment, the device includes a standard value computation module configured to obtain a current exercise intensity corresponding to the user to be assessed, and calculate a current oxygen saturation standard value based on the current exercise intensity and the current elevation. The determining module is further configured to determine the assessment result corresponding to the user to be assessed based on the current oxygen saturation, the first oxygen saturation, the current oxygen saturation prewarning value and the current oxygen saturation standard value.

In one embodiment, the standard value computation module is further configured to obtain a causation of elevation, exercise intensity and oxygen saturation standard value, the causation being obtained by analyzing oxygen saturation acquired under various elevations and exercise intensities, and calculate the current oxygen saturation standard value corresponding to the current exercise intensity and the current elevation based on the causation.

In one embodiment, the determining module is further configured to determine the assessment result corresponding to the user to be assessed as third adaptability level when the current oxygen saturation being greater than the current oxygen saturation prewarning value and the current oxygen saturation standard value being less than the current oxygen saturation prewarning value, and determine the assessment result corresponding to the user to be assessed as fourth adaptability level when the current oxygen saturation being greater than the current oxygen saturation prewarning value and the current oxygen saturation standard value being no less than the current oxygen saturation prewarning value.

In one embodiment, the device includes a climbing elevation computation module configured to obtain a predetermined oxygen saturation safety threshold and a predetermined exercise intensity, and calculate a climbable elevation corresponding to the user to be assessed based on the current oxygen saturation, the predetermined oxygen saturation safety threshold and the predetermined exercise intensity.

When executed by a processor, a computer readable storage medium, storing computer instructions, causing the processor to perform the following steps::
Obtaining an elevation and oxygen saturation corresponding to a user to be assessed;
Determining an assessment result corresponding to the user to be assessed based on the elevation and the oxygen saturation.

A smart wearable device, including a storage and a processor, the storage storing computer instructions, when executed by the processor, causes the processor to perform the following steps:
Obtaining an elevation and oxygen saturation corresponding to a user to be assessed;
Determining an assessment result corresponding to the user to be assessed based on the elevation and the oxygen saturation.

### The beneficial effects of the invention

### Beneficial effect

The oxygen saturation-based assessment method, device, smart wearable device and storage medium obtain the elevation and oxygen saturation corresponding to the user to be assessed, and then determine the assessment result corresponding to the user to be assessed based on the elevation and oxygen saturation. Based on the elevation and oxygen saturation, the plateau adaptability of the user to be assessed can be assessed timely, and thus the occurrence of plateau reaction and plateau sickness can be prevented in advance.

### BRIEF DESCRIPTION OF THE DRAWINGS

To describe technical solutions in embodiments of the present disclosure or in the related art more clearly, the following briefly introduces the accompanying drawings required for describing the embodiments or the related art. Apparently, the accompanying drawings in the following description illustrate merely some embodiments of the present disclosure. Those of ordinary skill in the art may also obtain other drawings based on these accompanying drawings without creative efforts.
FIG. 1 is a diagram of the application environment of the oxygen saturation-based assessment method in one embodiment;
FIG. 2 is a flowchart of an oxygen saturation-based assessment method in one embodiment;
FIG. 3 is a flowchart of an oxygen saturation-based assessment method in another embodiment;
FIG. 4 is a flowchart of an oxygen saturation-based assessment method in yet another embodiment;
FIG. 5 is a flowchart of a method of oxygen saturation-based assessment in yet another embodiment;
FIG. 6 is a diagram of the application of an oxygen saturation-based assessment method in one embodiment;
FIG. 7 is a schematic diagram of the structure of an pulse oximeter in one embodiment;
FIG. 8 is a schematic diagram of the structure of an pulse oximeter in another embodiment;
FIG. 9 is a block diagram of the structure of an oxygen saturation-based assessment device in one embodiment;
FIG. 10 is a block diagram of the structure of an oxygen saturation-based assessment device in another embodiment;
FIG. 11 shows a block diagram of an oxygen saturation-based assessment device in yet another embodiment;
FIG. 12 shows a block diagram of an oxygen saturation-based assessment device in yet another embodiment;
FIG. 13 shows a block diagram of the internal structure of a smart wearable device in one embodiment.

### DETAILED DESCRIPTION

In order to make the object, technical solutions and advantages of the present disclosure more clearly understood, the present disclosure is described in further detail hereinafter in conjunction with the accompanying drawings and embodiments. It should be understood that the embodiments described herein are intended to explain the disclosure only and are not intended to limit the disclosure.

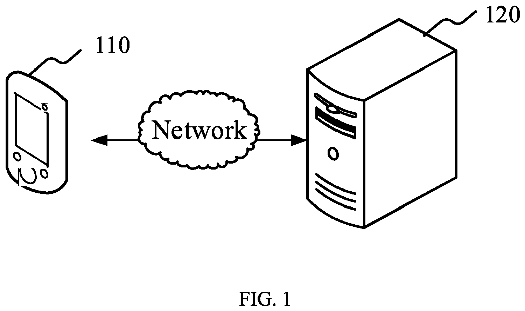

FIG. 1 shows a diagram of the application environment in which the oxygen saturation-based assessment method is applied in one embodiment. Referring to FIG. 1, the oxygen saturation-based assessment method may be applied to an oxygen saturation-based assessment system. The oxygen saturation-based assessment system includes a smart wearable device 110 and a server 120. The smart wearable device 110 and the server 120 are connected via a network. The smart wearable device 110 can be a smart watch, a smart helmet, a smart ring, etc. The server 120 may be implemented as a standalone server or a cluster of multiple servers. The smart wearable device 110 is used to obtain the elevation and oxygen saturation corresponding to the user to be assessed, upload the obtained elevation and oxygen saturation to the server 120, and the server 120 is used to obtain the elevation and oxygen saturation corresponding to the user to be assessed to determine the assessment result corresponding to the user to be assessed based on the elevation and oxygen saturation

In another embodiment, the oxygen saturation-based assessment method can be directly applied to the smart wearable device 110, the smart wearable device 110 is used to obtain the elevation and oxygen saturation corresponding to a user to be assessed, and determine the assessment result corresponding to the user to be assessed based on the elevation and oxygen saturation.

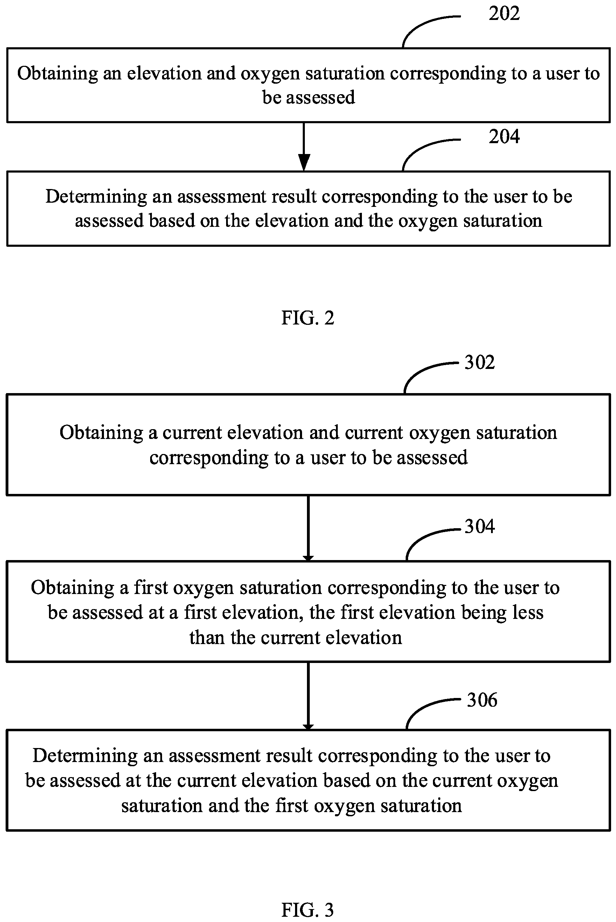

As shown in FIG. 2, an oxygen saturation-based assessment method is proposed, the method can be applied to a smart wearable device, a computer device connected to a smart wearable device, and a server, and this embodiment is illustrated by applying the oxygen saturation-based assessment method to a smart wearable device. The oxygen saturation-based assessment method may include the following steps:

Step 202, obtaining an elevation and oxygen saturation corresponding to a user to be assessed.

Among them, oxygen saturation (SpO2) is an important indicator of oxygen supply level of human body. The elevation and oxygen saturation of the user to be assessed are used to predict the user's plateau adaptability. The elevation can be measured by an elevation meter in a smart wearable device, or by GPS (Global Positioning System), or can be an elevation input by the user. Oxygen saturation can be measured by a pulse oximeter in the smart wearable device. The oxygen saturation can be measured in real time or at preset intervals. The obtained oxygen saturation can be the oxygen saturation corresponding to a current moment or the oxygen saturation of a previous moment.

In step 204, determining an assessment result corresponding to the user to be assessed based on the elevation and the oxygen saturation.

Among them, the assessment result can be an assessment of an obtained adaptability level (e.g., the plateau adaptability is divided into three levels to determine a current adaptability level corresponding to the user to be assessed), and can also be an assessment suggestion (e.g., a suggestion whether to continue climbing and a suggestion of climbable elevation), or can be an adaption score (e.g., a score corresponding to the user to be assessed can be generated on a ten-point scale or a percentile), and can be other assessment results that can be used to characterize the plateau adaptability.

In one embodiment, a current elevation and current oxygen saturation corresponding to the user to be assessed are obtained, standard oxygen saturation corresponding to the user to be assessed is obtained (e.g., the oxygen saturation at plain), and the assessment result corresponding to the user to be assessed is determined based on the current elevation, the current oxygen saturation, and the standard oxygen saturation.

In another embodiment, a rate of change of oxygen saturation can be calculated by obtaining the oxygen saturation of the user to be assessed over a certain period of time, and the assessment result of the user to be assessed may be determined based on the rate of change of oxygen saturation and the current oxygen saturation.

In one embodiment, first oxygen saturation at a first elevation and second oxygen saturation at a second elevation are obtained respectively, the rate of change of oxygen saturation is calculated based on the first oxygen saturation and the second oxygen saturation, and the assessment result of the user to be assessed can be determined based on the rate of change of oxygen saturation and the current oxygen saturation.

The above oxygen saturation-based assessment method obtains the elevation and the oxygen saturation corresponding to the user to be assessed, and then determines the assessment result corresponding to the user to be assessed based on the elevation and oxygen saturation. Based on the elevation and oxygen saturation, the plateau adaptability of the user to be assessed can be assessed timely, and thus the occurrence of plateau reaction and plateau sickness can be prevented in advance.

In one embodiment, obtaining the elevation and oxygen saturation corresponding to the user to be assessed, includes: obtaining a predicted elevation corresponding to the user to be assessed; determining the assessment result corresponding to the user to be assessed based on the elevation and the oxygen saturation, includes: calculating a rate of change of oxygen saturation based on the oxygen saturation corresponding to the user to be assessed; and determining the assessment result corresponding to the user to be assessed based on the predicted elevation and the rate of change of oxygen saturation.

Among them, the predicted elevation is the elevation at which the user to be assessed is to predict, i.e. the assessment result of plateau adaptability corresponding to the user to be assessed at that predicted elevation. In one embodiment, the oxygen saturation of the user to be assessed at different elevations is obtained, and then the rate of change of oxygen saturation is calculated based on the oxygen saturation corresponding to different elevations, a predicted oxygen saturation corresponding to the predicted elevation is calculated based on the rate of change of oxygen saturation and the predicted elevation, and the assessment result of the user to be assessed at that predicted elevation is determined based on the predicted oxygen saturation. The user to be assessed may input an elevation to which he/she wants to climb (e.g. the predicted elevation), and then calculate the assessment result of plateau adaptability corresponding to the user to be assessed at that predicted elevation based on the predicted elevation, so that subsequent exercises can be guided. In another embodiment, a rate of change of oxygen saturation can be statistically obtained directly from a smart wearable device and the oxygen saturation at an elevation can be obtained by the smart wearable device, the assessment result of plateau adaptability corresponding to a user to be assessed at the predicted elevation can be calculated based on the rate of change of oxygen saturation, the oxygen saturation at the elevation and the predicted elevation.

As shown in FIG. 3, an oxygen saturation-based assessment method is proposed, the method may include:
Step 302, obtaining a current elevation and current oxygen saturation corresponding to a user to be assessed.

Among them, oxygen saturation is an important indicator to indicate the oxygen supply level of human body. Plateau adaptability can be assessed by detecting the current oxygen saturation. The current elevation is the elevation at which the user to be assessed is currently located. The current oxygen saturation is the oxygen saturation of the user to be assessed that is currently detected. The current elevation and current oxygen saturation of the user (e.g., the user to be assessed) can be detected in real time by a smart wearable device. A pulse oximeter and an elevation meter may be installed on the smart wearable device, and the oxygen saturation (SpO2) is detected by the pulse oximeter and the current elevation is detected by the elevation meter.

Step 304, obtaining a first oxygen saturation corresponding to the user to be assessed at a first elevation, the first elevation is less than the current elevation.

Among them, the first elevation is an elevation that is less than the current elevation. In one embodiment, the first elevation may be an elevation at which oxygen saturation is at a steady state, i.e., a plain elevation. The first oxygen saturation is the oxygen saturation corresponding to the first elevation. It has been shown after a lot of research that the oxygen saturation fluctuates less when the elevation is below 2500 meters. Therefore, the first elevation can be set to any elevation less than 2500 meters, and the first oxygen saturation at the first elevation can be obtained by a smart wearable device.

Step 306, determining an assessment result corresponding to the user to be assessed at the current elevation based on the current oxygen saturation and the first oxygen saturation.

Among them, the assessment result of plateau adaptability is determined by comparing the current oxygen saturation with the first oxygen saturation. In one embodiment, when the difference between the first oxygen saturation and the current oxygen saturation is greater than a predetermined threshold, which indicates that the user to be assessed has poor plateau adaptability and continue climbing is not recommended. When the difference between the first oxygen saturation and the current oxygen saturation is not greater than the predetermined threshold, which indicates that the user to be assessed has a better plateau adaptability and can climb appropriately.

In another embodiment, a first predetermined threshold and a second predetermined threshold may be set, and the first predetermined threshold is greater than the second predetermined threshold. When the difference between the first oxygen saturation and the current oxygen saturation is greater than the first predetermined threshold, the plateau adaptability is set to level A accordingly; when the difference between the first oxygen saturation and the current oxygen saturation is not greater than the first predetermined threshold and is greater than the second predetermined threshold, the plateau adaptability is set to level B accordingly; when the difference between the first oxygen saturation and the current oxygen saturation is not greater than the second predetermined threshold, the plateau adaptability is set to level C accordingly. Level A indicates poor plateau adaptability and leaving the plateau is recommended, level B indicates average plateau adaptability and rest is recommended, level C indicates strong adaptability and continue climbing is recommended. Timely assessment of plateau adaptability based on current oxygen saturation and first oxygen saturation facilitates early prevention of plateau reactions.

In one embodiment, assessment result of plateau adaptability includes adaptability levels and corresponding recommendations. For example, if the assessment result is level A, the corresponding recommendation is included in the assessment result as well, for example, the recommendation may be leaving the plateau. By displaying assessment result of plateau adaptability on the smart wearable device, the user to be assessed can be reminded timely to prevent the occurrence of plateau reactions.

The above oxygen saturation-based assessment method, by detecting the current elevation and current oxygen saturation corresponding to the user to be assessed, obtains the first oxygen saturation corresponding to the user to be assessed at the first elevation, and determines the assessment result of plateau adaptability corresponding to the user to be assessed at the current elevation based on the current oxygen saturation and the first oxygen saturation. The current oxygen saturation and the first oxygen saturation can be used to assess the plateau adaptability of the user to be assessed timely, so that corresponding recommendations can be provided, which can prevent the occurrence of plateau reactions and plateau disease in advance and provide exercise guidance.

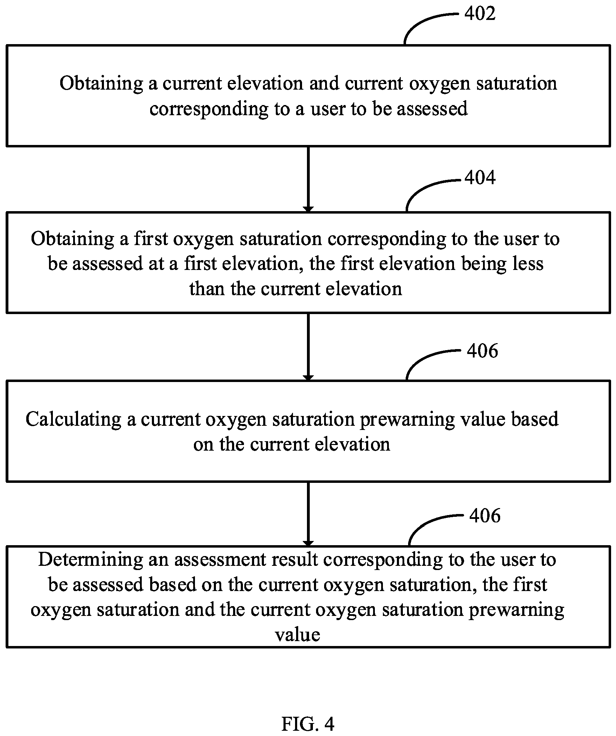

As shown in FIG. 4, in one embodiment, an oxygen saturation-based assessment method is proposed, the method may include.

Step 402, obtaining a current elevation and current oxygen saturation corresponding to a user to be assessed.

Step 404, obtaining a first oxygen saturation corresponding to the user to be assessed at a first elevation, the first elevation is less than the current elevation.

Step 406, calculating a current oxygen saturation prewarning value on the current elevation.

Among them, oxygen saturation prewarning value is an oxygen saturation value at which plateau reaction is likely to occur. Different elevations correspond to different oxygen saturation prewarning values, after obtaining the current elevation, the current oxygen saturation prewarning value corresponding to the current elevation is calculated.

Step 408, determining an assessment result corresponding to the user to be assessed based on the current oxygen saturation, the first oxygen saturation and the current oxygen saturation prewarning value.

Among them, the plateau adaptability of the user to be assessed is calculated by considering the current oxygen saturation, the first oxygen saturation and the current oxygen saturation prewarning value together. In one embodiment, when the difference between the first oxygen saturation and the current oxygen saturation is greater than a predetermined threshold (e.g., 30%), the assessment result of plateau adaptability is set to level I. When the current oxygen saturation is greater than the difference between the first oxygen saturation and the predetermined threshold and less than the current oxygen saturation prewarning value, the assessment result of plateau adaptability is set to level II. When the current oxygen saturation is not less than the current oxygen saturation prewarning value, then the assessment result of plateau adaptability is set to level III. Among them, the adaptability of level III > the adaptability of level II > the adaptability of level I.

In one embodiment, calculating the current oxygen saturation prewarning value based on the current elevation may include: obtaining a correlation between elevation and oxygen saturation prewarning value, the correlation is obtained by statistically analyzing the oxygen saturation corresponding to the occurrence of plateau reaction and non-plateau reaction at different elevations; calculating the current oxygen saturation prewarning value corresponding to the current elevation based on the correlation.

Among them, the correlation between elevation and oxygen saturation prewarning value may be preset. The correlation is obtained by statistically analyzing a large number of data of oxygen saturation corresponding to the occurrence of plateau reaction and non-plateau reaction at different elevations. In one embodiment, a nonlinear correlation between elevation and oxygen saturation prewarning value is obtained. The correlation may be expressed by using the following equation: f(x) = k1 + k2 ^{∗} x + k3 ^{∗} x^2. Among them, f(x) may denote oxygen saturation prewarning value, x may denote elevation, and k1, k2, k3 are constants. When the current elevation x is known, the current oxygen saturation prewarning value can be calculated. In one embodiment, the range of values of k1 is (90.07, 93.9), the range of values of k2 is (-0.0005465, 0.002189), and the range of values of k3 is (-9.549^{∗}10-7, -5.113^{∗}10-7).

In one embodiment, determining the assessment result of plateau adaptability corresponding to the user to be assessed based on the current oxygen saturation, the first oxygen saturation and the current oxygen saturation prewarning value may include: when the difference between the current oxygen saturation and the first oxygen saturation is greater than the predetermined threshold, then the assessment result corresponding to the user to be assessed is determined to be a first adaptability level; when the current oxygen saturation is less than the oxygen saturation prewarning value and greater than the difference between the first oxygen saturation and the predetermined threshold, then the assessment result corresponding to the user to be assessed is determined to be a second adaptability level.

Among them, preset the predetermined threshold, the predetermined threshold value can be empirically or statistically obtained. According to the conclusion of the literature, "the reduction of arterial oxygen saturation can predict the susceptibility of plateau pulmonary edema", a 30% reduction of oxygen saturation on the plateau in relative to plain can be used as an indicator to predict the risk of plateau pulmonary edema for people at risk of pulmonary edema, which has a universal application. Therefore, the predetermined threshold can be set to 30%. When the current oxygen saturation decreases by more than 30% relative to the first oxygen saturation, it is determined that the user to be assessed corresponds to the first adaptability level. When the current oxygen saturation is greater than the first oxygen saturation minus 30% and less than the oxygen saturation prewarning value, the corresponding assessment result is determined to be the second adaptability level. In one embodiment, the first adaptability level indicates very poor adaptability and leaving plateau is recommended. The second adaptability level indicates poor adaptability and rest is recommended.

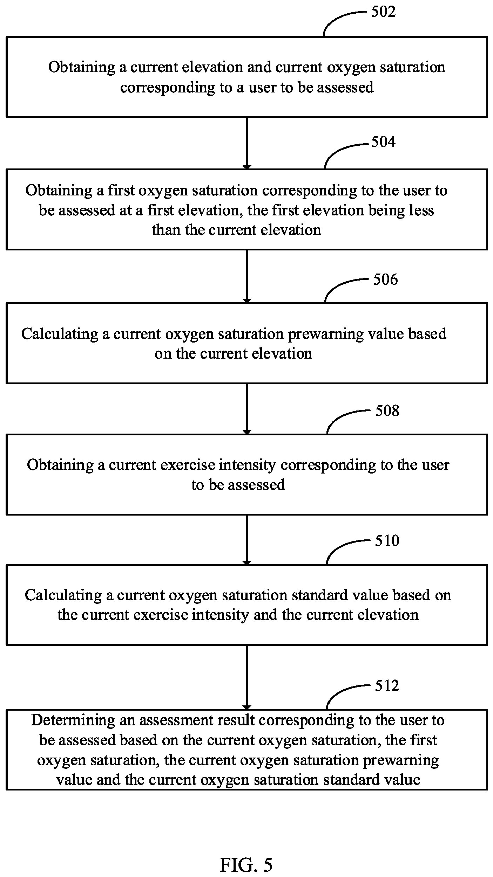

As shown in FIG. 5, in one embodiment, the above oxygen saturation-based assessment method may further include:
Step 502, obtaining a current elevation and current oxygen saturation corresponding to a user to be assessed.

Step 504, obtaining a first oxygen saturation of the user to be assessed at a first elevation, the first elevation being less than the current elevation.

Step 506, calculating a current oxygen saturation prewarning value based on the current elevation.

Step 508, obtaining a current exercise intensity corresponding to the user to be assessed.

Among them, the current exercise intensity indicates a current exercise state. The detected heart rate can be used to indicate the exercise intensity, and the faster the heart rate is, the higher the exercise intensity is.

Step 510, calculating a current oxygen saturation standard value based on the current exercise intensity and the current elevation.

Among them, oxygen saturation standard value is the normal value of oxygen saturation obtained statistically under different elevations and exercise intensities. Elevation and exercise intensity are two factors that affect oxygen saturation. At the same elevation, the higher the exercise intensity is, the more the oxygen saturation decreases; with the same exercise intensity and different elevations, the higher the elevation is, the more the oxygen saturation decreases. By obtaining the current exercise intensity and the current elevation, the current oxygen saturation standard value can be calculated.

Step 512, calculating an assessment result corresponding to the user to be assessed based on the current oxygen saturation, the first oxygen saturation, the current oxygen saturation prewarning value, the current oxygen saturation standard value.

Among them, the plateau adaptability of the user to be assessed is obtained by comparing the current oxygen saturation, the first oxygen saturation, the current oxygen saturation prewarning value, and the current oxygen saturation standard value. In one embodiment, when the current oxygen saturation is less than the difference between the first oxygen saturation and the predetermined threshold, it is set to level I. When the current oxygen saturation is not less than the difference between the first oxygen saturation and the predetermined threshold and is less than the current oxygen saturation prewarning value, it is set to level II, when the current oxygen saturation is greater than the current oxygen saturation prewarning value and less than the current oxygen saturation standard value, it is set to level III, and when the current oxygen saturation is greater than the current oxygen saturation standard value, it is set to level IV. Among them, the adaptability of level IV > the adaptability of level III > the adaptability of level II > the adaptability of level I.

In one embodiment, calculating the current oxygen saturation standard value based on the current exercise intensity and the current elevation may include: obtaining a causation between elevation, exercise intensity and oxygen saturation standard value, the causation is obtained by collecting and analyzing the oxygen saturation at different exercise intensities and elevations; calculating the current oxygen saturation standard value corresponding to the current exercise intensity and the current elevation based on the causation.

Among them, the correlation (i.e., causation) between elevation, exercise intensity and oxygen saturation standard value is calculated in advance. The causation is obtained by collecting a large number of oxygen saturation data corresponding to different exercise intensities at different elevations, and then conducting statistical analysis. In one embodiment, there is a nonlinear correlation between oxygen saturation standard value and elevation and exercise intensity. The elevation and exercise intensity are inversely correlated with the oxygen saturation standard value, respectively. That is, the higher the elevation, the lower the oxygen saturation standard value, and the higher the exercise intensity, the lower the oxygen saturation standard value. If the current exercise intensity and the current elevation are known, the current oxygen saturation standard value can be calculated based on this nonlinear correlation. In one embodiment, the following equation can be used to denote the relationship between elevation, exercise intensity and oxygen saturation standard value: g(x) = p1 + p2 ^{∗} x + p3 ^{∗} y +p4 ^{∗} x^ 2 + p5 ^{∗} x ^{∗} y. Among them, x may denote elevation, y may denote exercise intensity, and g(x) may denote oxygen saturation standard value. PI, p2, p3, p4 and p5 are the known coefficients. The range of values of p1 is (90.89, 105.7), the range of values of p2 is (-1.303, 6.345), the range of values of p3 is (-2.192, 1.412), the range of values of p4 is (-1.928, -0.7965), and the range of values of p5 is (-0.6572, 0.2704).

In one embodiment, calculating the assessment result of plateau adaptability corresponding to the user to be assessed based on the current oxygen saturation, the first oxygen saturation, the current oxygen saturation prewarning value, and the current oxygen saturation standard value includes: when the current oxygen saturation is greater than the current oxygen saturation prewarning value and the current oxygen saturation standard value is less than the current oxygen saturation prewarning value, the assessment result corresponding to the user to be assessed is determined as third adaptability level. When the current oxygen saturation is greater than the current oxygen saturation prewarning value and the current oxygen saturation standard value is not less than the current oxygen saturation prewarning value, the assessment result corresponding to the user to be assessed is determined as fourth adaptability level.

Among them, by comparing the current oxygen saturation, the current oxygen saturation prewarning value and the current oxygen saturation standard value, when the current oxygen saturation is greater than the current oxygen saturation prewarning value and the current oxygen saturation standard value is less than the current oxygen saturation prewarning value, then the plateau adaptability corresponding to the user to be assessed is determined as third adaptability level. When the current oxygen saturation is greater than the current oxygen saturation prewarning value, and the current oxygen saturation standard value is not less than the current oxygen saturation prewarning value, the plateau adaptability corresponding to the user to be assessed is determined as fourth adaptability level. Third adaptability level indicates general adaptability, rest is recommended and appropriate adaptability activities can be carried out, but no strenuous exercise. Fourth adaptability level indicates good adaptability, and it is recommended that climbing can be continued at an appropriate exercise intensity.

In one embodiment, the above oxygen saturation-base assessment method may further include: obtaining a predetermined oxygen saturation safety threshold and a predetermined exercise intensity; calculating a climbable elevation corresponding to the user to be assessed based on the current oxygen saturation, the oxygen saturation safety threshold and the predetermined exercise intensity.

Among them, the oxygen saturation safety threshold value is the minimum safety value of oxygen saturation that will not cause plateau reaction. Oxygen saturation safety threshold can be preset or oxygen saturation prewarning value can be used as oxygen saturation safety threshold. The preset exercise intensity can be one or more. Different exercise intensities correspond to different climbable elevations. In one embodiment, the current exercise intensity can also be directly used as the preset exercise intensity. Based on the current oxygen saturation, the oxygen saturation safety threshold and the preset exercise intensity, the climbable elevation of the user to be assessed under the preset exercise intensity can be assessed. The user to be assessed is then given the corresponding climbing recommendation. In one embodiment, a specific recommended climbable elevation is given when the assessment result of plateau adaptability is good. The climbable elevation can be calculated using the following formula, h(x) = ax + bx^2 + c ^{∗} xy, among them, h(x) may denote the decreasing value of oxygen saturation, x may denote climbable elevation, y may denote preset exercise intensity, and a, b and c may denote constants. The decreasing value of oxygen saturation can be calculated based on the current oxygen saturation and the oxygen saturation safety threshold value, and the climbable elevation can be calculated if h(x) and y are known.

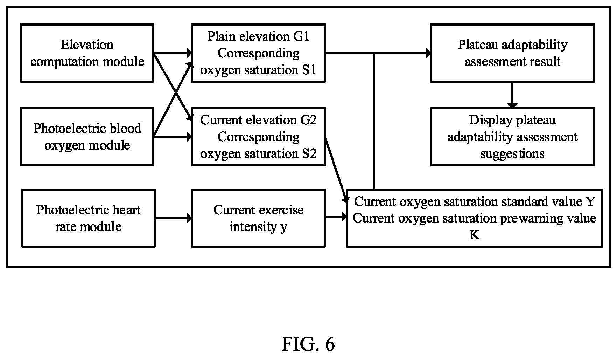

As shown in Figure 6, a schematic diagram of one embodiment in which the oxygen saturation-based assessment method is applied. As shown in Figure 6, a smart wearable device (e.g., a smart wearable watch) may include an altimeter module, a photoelectric heart rate module, and a photoelectric oxygen module (e.g., pulse oximeter), the altimeter module is used to measure elevation and the photoelectric heart rate module is used to measure heart rate, i.e., exercise intensity. The photoelectric oxygen module is used to measure oxygen saturation. Firstly, the altimeter module measures a plain elevation G1 and oxygen saturation S1 is detected by the photoelectric oxygen module when it is on a plain (below 2500m), and when it reaches a plateau (above 2500m), it measures a current elevation G2 by the altimeter module in real-time, a current exercise intensity y obtained by the photoelectric heart rate module, a current oxygen saturation S2 by the photoelectric oxygen module, a current oxygen saturation standard value Y may be calculated based on the current exercise intensity and the current elevation, and a current oxygen saturation prewarning value K may be calculated based on the current elevation. By comparing S1, S2, K and Y, the corresponding adaptability level of the user to be assessed is calculated, and then the corresponding recommendation is given according to the adaptability level and displayed on the smart wearable device.

In one embodiment, when S1 is less than S2-30%, it indicates that the adaptability level is grade A, and the oxygen saturation range of level A is between (64%-70%); when S1 is greater than S2-30% and less than the value of K, it indicates that the adaptability level is level B, and the oxygen saturation range of grade B is between (64%-85%); when S1 is greater than K, and K is greater than the value of Y indicates that the adaptability level is level C, and the oxygen saturation range of grade C ranges from (70%-90%); when S1 is greater than K, and K is not-greater than Y, it indicates that the adaptability level is grade D, and the oxygen saturation range of level D ranges from (75%-94%).

In one embodiment, the smart wearable device includes a pulse oximeter as well as a pressure sensor, the pulse oximeter and the pressure sensor are provided on a device such as a smart watch or a head wearable device. Among them the pulse oximeter can be used to detect the oxygen saturation of the wearer. The pressure sensor may be used to detect the wearing pressure of the wearer while wearing the wearable device, for example, to detect the magnitude of the pressure of the strap on the wrist of the wearer while wearing the smart watch, as another example, to detect the magnitude of the pressure of the head wearable device on the head of the wearer while wearing the head wearable device.

In one embodiment, the pulse oximeter may further include an infrared light emitting unit, a red light emitting unit, and a light detecting unit; emitting infrared light to an object through the infrared light emitting unit, and emitting red light to the object through the red light emitting unit; receiving the light signal corresponding to the infrared light and the red light reflected from the object through the light detecting unit, and converting the light signal into an electrical signal through a photoelectric receiver; calculating a first ratio of the AC amplitude to the DC amplitude of the red light signal received by the light detection unit, calculating a second ratio of the AC amplitude to the DC amplitude of the infrared light signal received by the light detection unit, and determining an initial oxygen saturation by calculating the ratio of the first ratio to the second ratio. Detecting a wear pressure value of the smart wearable device during wearing by the pressure sensor as a target pressure value; filtering the initial oxygen saturation according to a preset pressure calibration data model and the target pressure value to obtain a corrected oxygen saturation corresponding to the initial oxygen saturation.

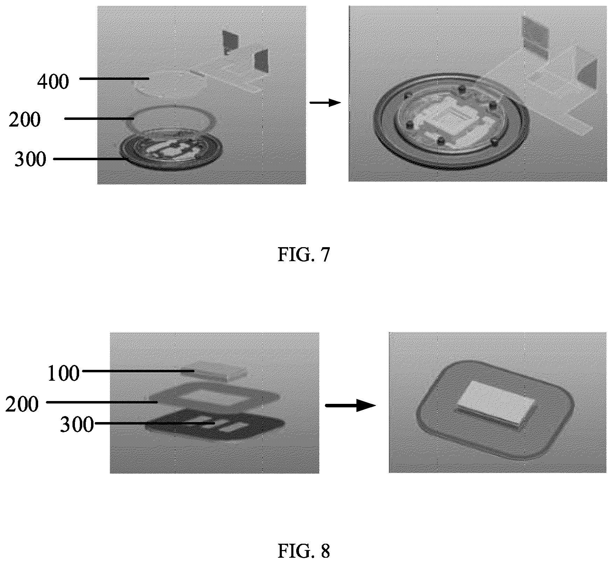

In a specific embodiment, an example of an installation of a pressure sensor 200 in a specific embodiment is given as shown in FIGs. 7 and 8, where the pressure sensor 200 is fixed to a housing 300, where the pressure is detected by the deformation of the housing 300, and where the housing 300 has an aperture for light to pass through. Specifically, as shown in FIG. 5, the pressure sensor 200 is circular, a pulse oximeter 100 is set on a mounting plate 400, and then the mounting plate 400, the pressure sensor 200, and the housing 300 are fixed together; as shown in FIG. 6, the pressure sensor is rectangle 200, and the pressure sensor is fixed together with the pulse oximeter 100 and the housing 300.

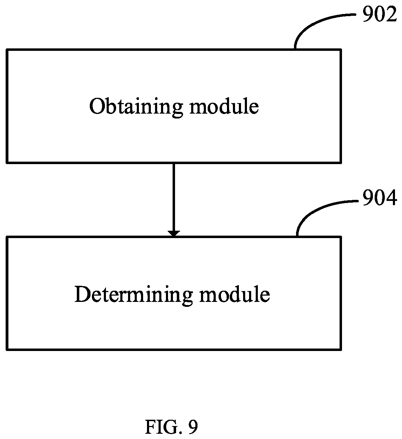

As shown in Figure 9, an oxygen saturation-based assessment device is proposed, the oxygen saturation-based assessment device may include:

An acquisition module 902 for obtaining an elevation and oxygen saturation corresponding to a user to be assessed.

A determining module 904 for determining an assessment result corresponding to the user to be assessed based on the elevation and the oxygen saturation.

In one embodiment, the obtaining module 902 may include: an elevation meter and a pulse oximeter, elevation is measured by the elevation meter and oxygen saturation is measured by the pulse oximeter; the determining module 904 may be a processor, the processor may calculate the assessment result corresponding to the user to be assessed based on the elevation and the oxygen saturation.

In one embodiment, the obtaining module is further used to obtain a predicted elevation corresponding to the user to be assessed; the determining module is further used to calculate a rate of change of oxygen saturation based on the oxygen saturation corresponding to the user to be assessed; and to determine the assessment result corresponding to the user to be assessed based on the predicted elevation and the rate of change of oxygen saturation.

In one embodiment, the obtaining module is further used to obtain a current elevation and current oxygen saturation corresponding to the user to be assessed, to obtain a first oxygen saturation corresponding to the user to be assessed at a first elevation, the first elevation is less than the current elevation; the determining module is further used to determine an assessment result corresponding to the user to be assessed at the current elevation based on the current oxygen saturation and the first oxygen saturation.

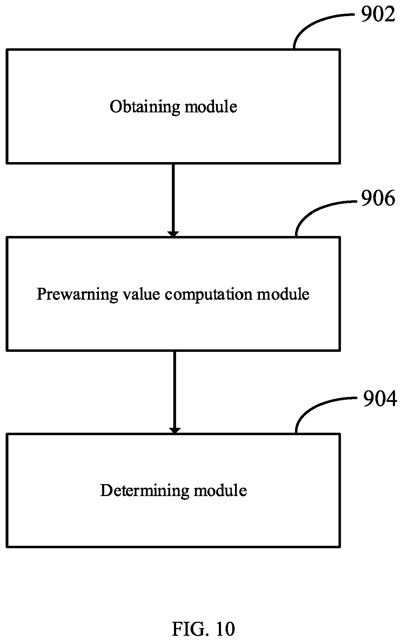

As shown in FIG. 10, in one embodiment, the oxygen saturation-based assessment device may further include:
A prewarning value computation module 906 for calculating a current oxygen saturation prewarning value based on the current elevation; the determining module is further used to determine an assessment result corresponding to the user to be assessed based on the current oxygen saturation, the first oxygen saturation and the current oxygen saturation prewarning value.

In one embodiment, the prewarning value computation module is further used to obtain a correlation between elevation and oxygen saturation prewarning value, the correlation is obtained by statistically analyzing the oxygen saturation corresponding to the occurrence of plateau reactions and non- plateau reactions at different elevations; the current oxygen saturation prewarning value corresponding to the current elevation is calculated based on the correlation.

In one embodiment, the determining module is further used to determine that the assessment result corresponding to the user to be assessed as a first adaptability level when the difference between the current oxygen saturation and the first oxygen saturation is greater than a predetermined threshold; and to determine that the assessment result corresponding to the user to be assessed as a second adaptability level when the current oxygen saturation is less than the oxygen saturation prewarning value and greater than the difference between the first oxygen saturation and the predetermined threshold.

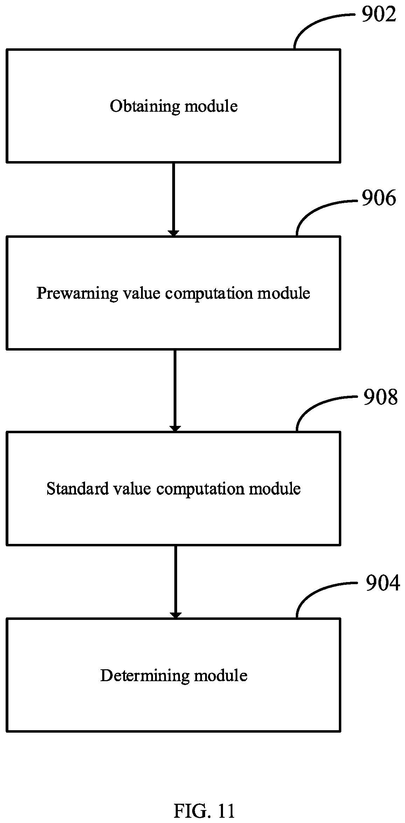

As shown in FIG. 11, in one embodiment, the oxygen saturation-based assessment device may further include:
A standard value computation module 908 for obtaining a current exercise intensity corresponding to the user to be assessed and calculating a current oxygen saturation standard value based on the current exercise intensity and the current elevation.

The determining module is further used to obtain an assessment result corresponding to the user to be assessed based on the current oxygen saturation, the first oxygen saturation, the current oxygen saturation prewarning value, and the current oxygen saturation standard value.

In one embodiment, the standard value computation module is further used to obtain a causation between elevation, exercise intensity and oxygen saturation standard value. The causation is obtained by collecting and analyzing oxygen saturation at different exercise intensities at different elevations. A current oxygen saturation standard value corresponding to the current exercise intensity and the current elevation is calculated according to the causation.

In one embodiment, the determining module is further used to determine that when the current oxygen saturation is greater than the current oxygen saturation prewarning value and the current oxygen saturation standard value is less than the current oxygen saturation prewarning value, the corresponding assessment result for the user to be assessed is a third adaptability level; when the current oxygen saturation is greater than the current oxygen saturation prewarning value and the current oxygen saturation standard value is not less than the current oxygen saturation prewarning value, the corresponding assessment result for the user to be assessed is determined to be a fourth adaptability level.

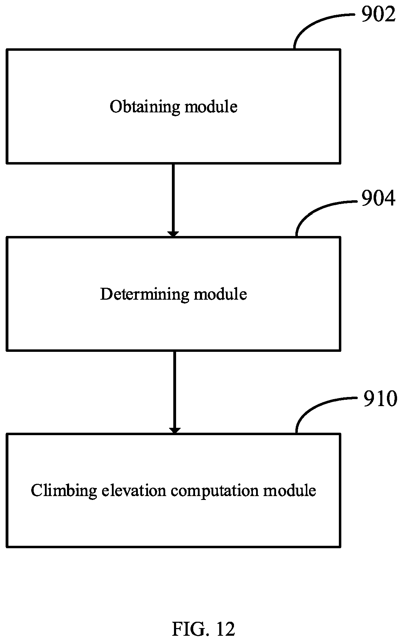

As shown in FIG. 12, in one embodiment, the oxygen saturation-based assessment device may further include:
A climbing elevation computation module 910 for obtaining a predetermined oxygen saturation safety threshold and a predetermined exercise intensity and calculating a climbable elevation corresponding to the user to be assessed based on the current oxygen saturation, the predetermined oxygen saturation safety threshold and the predetermined exercise intensity.

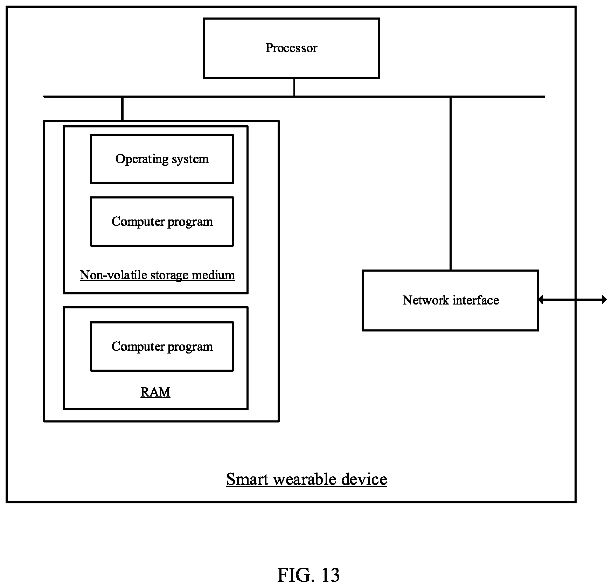

FIG. 13 illustrates a block diagram of a smart wearable device in one embodiment. The smart wearable device may be a smart wearable watch, a smart wearable helmet, a smart wearable ring, etc. As shown in FIG. 13, the smart wearable device includes a processor, a memory, and a network interface connected via a system bus. Among them, the memory may include a non-volatile storage medium and a random-access memory (RAM). The non-volatile storage medium of the smart wearable device may store an operating system and may also store computer instructions that, when executed by the processor, may cause the processor to perform an oxygen saturation-based assessment method. The random-access memory may also store computer instructions that, when executed by the processor, may cause the processor to perform the oxygen saturation-based assessment method. It will be understood by those skilled in the art that the structure illustrated in FIG. 13, which is only a block diagram of a portion of the structure associated with the present application solution, does not constitute a limitation of the smart wearable device to which the present application solution is applied, and that a specific smart wearable device may include more or less components than shown in the figure, or a combination of certain components, or have a different arrangement of components.

In one embodiment, the oxygen saturation-based assessment method provided by the present application may be implemented in the form of computer instructions, and the computer instructions may run on a smart wearable device as shown in FIG. 13. The various program modules comprising the oxygen saturation-based assessment device may be stored in the memory of the smart wearable device. For example, the acquisition module 902 and the determining module 904 in FIG. 9.

In one embodiment, a smart wearable device is disclosed comprising a memory and a processor, the memory may store computer instructions, the computer instructions, when executed by the processor, causing the processor to perform the steps of: obtaining an elevation and oxygen saturation corresponding to a user to be assessed; determining an assessment result corresponding to the user to be assessed based on the elevation and the oxygen saturation.

In one embodiment, obtaining the elevation and oxygen saturation corresponding to the user to be assessed may include: obtaining a predicted elevation corresponding to the user to be assessed; determining an assessment result corresponding to the user to be assessed based on the elevation and the oxygen saturation may include: calculating a rate of change of oxygen saturation based on the oxygen saturation corresponding to the user to be assessed; and determining the assessment result corresponding to the user to be assessed based on the predicted elevation and the rate of change of oxygen saturation.

In one embodiment, obtaining the elevation and oxygen saturation corresponding to the user to be assessed may include: obtaining a current elevation and a current oxygen saturation corresponding to the user to be assessed; obtaining a first oxygen saturation corresponding to the user to be assessed at a first elevation, the first elevation is less than the current elevation; determining the assessment result corresponding to the user to be assessed based on the elevation and the oxygen saturation may include: determining the assessment result corresponding to the user to be assessed at the current elevation based on the current oxygen saturation and the first oxygen saturation.

In one embodiment, the method may further include calculating a current oxygen saturation prewarning value based on the current elevation; determining an assessment result corresponding to the user to be assessed at the current elevation based on the current oxygen saturation and the first oxygen saturation may include: determining the assessment result corresponding to the user to be assessed at the current elevation based on the current oxygen saturation, the first oxygen saturation and the current oxygen saturation prewarning value.

In one embodiment, calculating the current oxygen saturation prewarning value based on the current elevation may include: obtaining a correlation between elevation and oxygen saturation prewarning value, the correlation is obtained by statistically analyzing the oxygen saturation corresponding to the occurrence of plateau reaction and non-plateau reactions at different elevations; calculating the current oxygen saturation prewarning value corresponding to the current elevation based on the correlation.

In one embodiment, determining the assessment result corresponding to the user to be assessed based on the current oxygen saturation, the first oxygen saturation and the current oxygen saturation prewarning value may include: when the difference between the current oxygen saturation and the first oxygen saturation is greater than a predetermined threshold, then determining that the assessment result corresponding to the user to be assessed as a first adaptability level; when the current oxygen saturation is less than the oxygen saturation prewarning value and greater than the difference between the first oxygen saturation and the predetermined threshold, then determining that the assessment result corresponding to the user to be assessed as a second adaptability level.

In one embodiment, the method may further include: obtaining a current exercise intensity corresponding to the user to be assessed; calculating a current oxygen saturation standard value based on the current exercise intensity and the current elevation; determining the assessment result corresponding to the user to be assessed based on the current oxygen saturation, the first oxygen saturation and the current oxygen saturation prewarning value, may include: calculating the assessment result corresponding to the user to be assessed based on the current oxygen saturation, the first oxygen saturation, the current oxygen saturation prewarning value and the current oxygen saturation standard value.

In one embodiment, calculating the current oxygen saturation standard value based on the current exercise intensity and the current elevation may include: obtaining a causation between elevation, exercise intensity and oxygen saturation standard value, the causation is obtained by collecting and analyzing oxygen saturation at different exercise intensities at different elevations; calculating the current oxygen saturation standard value corresponding to the current exercise intensity and the current elevation based on the causation.

In one embodiment, calculating the assessment result corresponding to the user to be assessed based on the current oxygen saturation, the first oxygen saturation, the current oxygen saturation prewarning value, the current oxygen saturation standard value may include: when the current oxygen saturation is greater than the current oxygen saturation prewarning value and the current oxygen saturation standard value is less than the current oxygen saturation prewarning value, determining that the user to be assessed corresponds to an assessment result of a third adaptability level; when the current oxygen saturation is greater than the current oxygen saturation prewarning value and the current oxygen saturation standard value is not less than the current oxygen saturation prewarning value, determining that the user to be assessed corresponds to an assessment result of a fourth adaptability level.

In one embodiment, the computer instructions, when executed by the processor, further causes the processor to perform the steps of: obtaining a predetermined oxygen saturation safety threshold value and a predetermined exercise intensity; and calculating a climbable elevation corresponding to the user to be assessed based on the current oxygen saturation, the oxygen saturation safety threshold value and the predetermined exercise intensity.

In one embodiment, a computer readable storage medium is disclosed, the computer readable storage medium stores computer instructions, when executed by a processor, may cause the processor to perform the steps of: obtaining an elevation and oxygen saturation corresponding to a user to be assessed; determining an assessment result corresponding to the user to be assessed based on the elevation and the oxygen saturation.

In one embodiment, obtaining the elevation and oxygen saturation corresponding to the user to be assessed may include: obtaining a predicted elevation corresponding to the user to be assessed; determining an assessment result corresponding to the user to be assessed based on the elevation and the oxygen saturation may include: calculating a rate of change of oxygen saturation based on the oxygen saturation corresponding to the user to be assessed; and determining the assessment result corresponding to the user to be assessed based on the predicted elevation and the rate of change of oxygen saturation.

In one embodiment, obtaining the elevation and oxygen saturation corresponding to the user to be assessed may include: obtaining a current elevation and a current oxygen saturation corresponding to the user to be assessed; obtaining a first oxygen saturation corresponding to the user to be assessed at a first elevation, the first elevation is less than the current elevation; determining the assessment result corresponding to the user to be assessed based on the elevation and the oxygen saturation may include: determining the assessment result corresponding to the user to be assessed at the current elevation based on the current oxygen saturation and the first oxygen saturation.

In one embodiment, the method may further includes calculating a current oxygen saturation prewarning value based on the current elevation; determining an assessment result corresponding to the user to be assessed at the current elevation based on the current oxygen saturation and the first oxygen saturation may include: determining the assessment result corresponding to the user to be assessed at the current elevation based on the current oxygen saturation, the first oxygen saturation and the current oxygen saturation prewarning value.

In one embodiment, calculating the current oxygen saturation prewarning value based on the current elevation may include: obtaining a correlation between elevation and oxygen saturation prewarning value, the correlation is obtained by statistically analyzing the oxygen saturation corresponding to the occurrence of plateau reaction and non- plateau reaction at different elevations; calculating the current oxygen saturation prewarning value corresponding to the current elevation based on the correlation.

In one embodiment, determining the assessment result corresponding to the user to be assessed based on the current oxygen saturation, the first oxygen saturation and the current oxygen saturation prewarning value may include: when the difference between the current oxygen saturation and the first oxygen saturation is greater than a predetermined threshold, then determining that the assessment result corresponding to the user to be assessed as a first adaptability level; when the current oxygen saturation is less than the oxygen saturation prewarning value and greater than the difference between the first oxygen saturation and the predetermined threshold, then determining that the assessment result corresponding to the user to be assessed as a second adaptability level.

In one embodiment, the method may further include: obtaining a current exercise intensity corresponding to the user to be assessed; calculating a current oxygen saturation standard value based on the current exercise intensity and the current elevation; determining the assessment result corresponding to the user to be assessed based on the current oxygen saturation, the first oxygen saturation and the current oxygen saturation prewarning value, may include: calculating the assessment result corresponding to the user to be assessed based on the current oxygen saturation, the first oxygen saturation, the current oxygen saturation prewarning value and the current oxygen saturation standard value.

In one embodiment,-calculating the current oxygen saturation standard value based on the current exercise intensity and the current elevation may include: obtaining a causation between elevation, exercise intensity and oxygen saturation standard value, the causation is obtained by collecting and analyzing oxygen saturation at different exercise intensities at different elevations; calculating the current oxygen saturation standard value based on the causation.

In one embodiment, calculating the assessment result corresponding to the user to be assessed based on the current oxygen saturation, the first oxygen saturation, the current oxygen saturation prewarning value, the current oxygen saturation standard value may include: when the current oxygen saturation is greater than the current oxygen saturation prewarning value and the current oxygen saturation standard value is less than the current oxygen saturation prewarning value, determining that the user to be assessed corresponds to an assessment result of a third adaptability level; when the current oxygen saturation is greater than the current oxygen saturation prewarning value and the current oxygen saturation standard value is not less than the current oxygen saturation prewarning value, determining that the user to be assessed corresponds to an assessment result of a fourth adaptability level.

In one embodiment, the computer instructions, when executed by the processor, may further cause the processor to perform the steps of: obtaining a predetermined oxygen saturation safety threshold value and a predetermined exercise intensity; and calculating a climbable elevation corresponding to the user to be assessed based on the current oxygen saturation, the oxygen saturation safety threshold value and the predetermined exercise intensity.

A person of ordinary skill in the art can understand that achieving all or part of the processes in the methods of the above embodiments is possible by means of a computer instructions to instruct the relevant hardware to do so, and the program can be stored in a non-volatile computer readable storage medium which, when the program executed, can include processes such as those of the embodiments of the respective methods described above. Among them, any reference to memory, storage, database, or other media used in the embodiments provided in this application may include non-volatile and/or volatile memory. The non-volatile memory may include read-only memory (ROM), programmable ROM (PROM), electrically programmable ROM (EPROM), electrically erasable programmable ROM (EEPROM), or flash memory. Volatile memory may include random access memory (RAM) or external cache memory. By way of illustration and not limitation, RAM is available in a variety of forms, such as static RAM (SRAM), dynamic RAM (DRAM), synchronous DRAM (SDRAM), double data rate SDRAM (DDRSDRAM), enhanced SDRAM (ESDRAM), synchronous link (Synchlink) DRAM (SLDRAM), memory Bus (Rambus) Direct RAM (RDRAM), Direct Memory Bus Dynamic RAM (DRDRAM), and Memory Bus Dynamic RAM (RDRAM), etc.

Each technical feature of the above-described embodiments can be combined in various ways. For the sake of brevity of description, not all possible combinations of each technical feature of the above-described embodiments are been described; however, as long as there is no contradiction in the combination of these technical features, they should be considered to be within the scope of the present specification.

The above described embodiments merely discloses several implementations of the present application, and their descriptions are more specific and detailed, but they should not be construed as a limitation of the scope of the patent of the present application. It should be noted that for a person of ordinary skill in the art, without departing from the conception of the present application, a number of variations and improvements can be made, which all belong to the scope of protection of the present application. Therefore, the scope of protection of the patent of this application shall be subject to the attached claims.

## Claims

1. An oxygen saturation-based assessment method, the method comprising:
obtaining an elevation and oxygen saturation corresponding to a user to be assessed;
determining an assessment result corresponding to the user to be assessed based on the elevation and the oxygen saturation.

2. The method of claim 1, wherein obtaining the elevation and the oxygen saturation corresponding to the user to be assessed comprising:
obtaining a predicted elevation corresponding to the user to be assessed;
wherein determining the assessment result corresponding to the user to be assessed based on the elevation and the oxygen saturation comprising:
calculating a rate of change of oxygen saturation based on the oxygen saturation corresponding to the user to be assessed;
determining the assessment result corresponding to the user to be assessed based on the predicted elevation and the rate of change of oxygen saturation.

3. The method of claim 1, wherein obtaining the elevation and the oxygen saturation corresponding to the user to be assessed comprising:
obtaining a current elevation and a current oxygen saturation corresponding to the user to be assessed;
obtaining a first oxygen saturation corresponding to the user to be assessed at a first elevation, the first elevation being less than the current elevation;
wherein determining the assessment result corresponding to the user to be assessed based on the elevation and the oxygen saturation comprising:
determining the assessment result corresponding to the user to be assessed at the current elevation based on the current oxygen saturation and the first oxygen saturation.

4. The method of claim 3, the method further comprising:
calculating a current oxygen saturation prewarning value based on the current elevation;
wherein determining the assessment result corresponding to the user to be assessed at the current elevation based on the current oxygen saturation and the first oxygen saturation comprising:
determining the assessment result corresponding to the user to be assessed based on the current oxygen saturation, the first oxygen saturation and the current oxygen saturation prewarning value.

5. The method of claim 4, where calculating the current oxygen saturation prewarning value based on the current elevation comprising:
obtaining a correlation between elevation and oxygen saturation prewarning value, the correlation being obtained based on the oxygen saturation corresponding to plateau reaction and non-plateau reaction under different elevations;
calculating the current oxygen saturation prewarning value corresponding to the current elevation based on the correlation.

6. The method of claim 4, wherein determining the assessment result corresponding to the user to be assessed based on the current oxygen saturation, the first oxygen saturation and the current oxygen saturation prewarning value comprising:
determining the assessment result corresponding to the user to be assessed as first adaptability level when the difference of the current oxygen saturation and the first oxygen saturation being greater than a predetermined threshold;
determining the assessment result corresponding to the user to be assessed as second adaptability level when the current oxygen saturation being less than the current oxygen saturation prewarning value and greater than the difference of the first oxygen saturation and the predetermined threshold.

7. The method of claim 4, the method further comprising:
obtaining a current exercise intensity corresponding to the user to be assessed;
calculating a current oxygen saturation standard value based on the current exercise intensity and the current elevation;
wherein determining the assessment result corresponding to the user to be assessed based on the current oxygen saturation, the first oxygen saturation and the current oxygen saturation prewarning value comprising:
determining the assessment result corresponding to the user to be assessed based on the current oxygen saturation, the first oxygen saturation, the current oxygen saturation prewarning value and the current oxygen saturation standard value.

8. The method of claim 7, wherein calculating the current oxygen saturation standard value based on the current exercise intensity and the current elevation comprising:
obtaining a causation of elevation, exercise intensity and oxygen saturation standard value, the causation being obtained by analyzing oxygen saturation acquired under various elevations and exercise intensities;
calculating the current oxygen saturation standard value corresponding to the current exercise intensity and the current elevation based on the causation.

9. The method of claim 7, wherein determining the assessment result corresponding to the user to be assessed based on the current oxygen saturation, the first oxygen saturation, the current oxygen saturation prewarning value and the current oxygen saturation standard value comprising:
determining the assessment result corresponding to the user to be assessed as third adaptability level when the current oxygen saturation being greater than the current oxygen saturation prewarning value and the current oxygen saturation standard value being less than the current oxygen saturation prewarning value;
determining the assessment result corresponding to the user to be assessed as fourth adaptability level when the current oxygen saturation being greater than the current oxygen saturation prewarning value and the current oxygen saturation standard value being no less than the current oxygen saturation prewarning value.

10. The method of claim 1, the method further comprising:
obtaining a predetermined oxygen saturation safety threshold and a predetermined exercise intensity;
calculating a climbable elevation corresponding to the user to be assessed based on the current oxygen saturation, the predetermined oxygen saturation safety threshold and the predetermined exercise intensity.

11. An on oxygen saturation-based assessment device, comprising:
an obtaining module configured to obtain an elevation and oxygen saturation corresponding to a user to be assessed;
a determining module configured to determine an assessment result corresponding to the user to be assessed based on the elevation and the oxygen saturation.

12. A computer readable medium, storing computer instructions, when executed by a processor, causes the processor to:
perform the method as claimed in any of claims 1-10.

13. A smart wearable device, including a storage and a processor, the storage storing computer instructions, when executed by the processor, causes the processor to:
perform the method as claimed in any of claims 1-10.
